# EUROPEAN PATENT SPECIFICATION

(11) **EP 1 656 440 B1**
(45) Date of publication and mention of the grant of the patent: **23.01.2008**
(21) Application number: 04744256.1
(22) Date of filing: 12.08.2004
(51) Int. Cl.: C11D 3/50, C11D 17/04

(54) **PACKAGED PRODUCT**
PACKGUT
PRODUIT CONDITIONNE

(30) Priority: 13.08.2003 WO PCT/IB03/03601
(43) Date of publication of application: 17.05.2006
(73) Proprietor: FIRMENICH SA, 1211 Genève 8 (CH)
(72) Inventor: HOLZNER, Günter, CH-1212 Grand-Lancy (CH); VERHOVNIK, Glenn, CH-1224 Chêne-Bougeries (CH)
(74) Representative: Salvaterra-Garcia, Maria de Lurdes
(86) International application number: PCT/IB2004/002622
(87) International publication number: WO 2005/017085

(56) References cited:
- EP-A- 0 612 843
- WO-A-02/074430
- US-A1- 2003 134 765

## Description

### Technical Field

The present invention relates to the perfume industry. It concerns more particularly a packaged product comprising, in a container, a liquid base containing a surfactant system, and a perfuming base comprising fragranced aminoplast microcapsules, namely melamine resin microcapsules, or fragranced coacervate microcapsules. The invention is characterised by the fact that the container comprises at least two physically separated compartments and that the surfactant-containing liquid base on the one hand, and the fragranced capsule base on the other hand, are held in separate compartments.

### Background Art

Melamine resins and urea resins constitute the most important representatives of the amino resins. They result from a kind of Mannich reaction between NH-containing compounds, nucleophilic molecules and carbonyl-containing compounds. NH-Components are mainly urea H₂N-CO-NH₂ or melamine (2,4,6-triamino-1,3,5-triazine). The carbonyl component is predominantly formaldehyde (rarely ketones or other aldehydes). Nucleophilic components may be H-acidic (halogen acids), OH compounds (alcohols, carboxylic acids), or NH compounds (urea, melamine, amines etc.) The resulting "aminoplastics" are colourless. Melamine resins are used for shatter-proof tableware. Moreover, the literature reports the use of amino resins for the encapsulation of active substances and mentions in particular the potential use of such encapsulation systems in perfumery and cosmetic applications. Therefore, amino resin based capsules, also commonly designated as aminoplast capsules, are the subject of a variety of literature reports and patent applications relating to the perfumery and cosmetic industries. In practice, these polymers are capable of forming a protecting shell around the active ingredient that one wishes to protect, thus providing an encapsulation system characterised by its water-insolubility. The active ingredient protected by the capsule may be released through mechanical rupture of the brittle microcapsules.

Now, while capsules based on the use of these polymers provide a good way of protecting volatile and labile ingredients such as perfuming compounds from degradation in certain media, for instance in an aqueous environment, they cannot be used in functional consumer products wherein the treating base of the product comprises a surfactant system, as they turn out to be unstable in such a medium. More particularly, it is known in the art that even if the shell of such microcapsules is not soluble in water, a cleaning solution such as a detergent solution, which contains surfactant compounds, washes out the fragrance contained in the capsules and leaves an empty shell after some weeks. Therefore, this type of chemically reactive aminoplast microcapsules cannot be used in consumer products containing a surfactant system.

Another type of microcapsules encounters the same problematic and is therefore submitted to the same limitation in its use. These are coacervate systems. Coacervation, also called aqueous phase separation, is a very well known technique for encapsulating hydrophobic liquids. A coacervation process allows to provide oil-containing microscopic or larger capsules, the encapsulating material being a gelled hydrophilic colloid that is impervious to the oil and deposited evenly and densely around the oil forming the nucleus. The encapsulating colloid material is a protein which may be complexed with another colloid having an opposite charge. Now, the products of simple or complex coacervation processes are unstable in a surfactant-containing environment, and this prevents their use in most consumer products of the detergent or cleaning type.

The process for the preparation of aminoplast microcapsules containing encapsulated fragrance is a well-known state of the art and is described in the patent literature, for example in US Patent 3,516,941, US Patent 4,976,961, DE Patent 198,33,347 (BASF), WO Patent 01/51197 (BASF) and US Patent 6,261,483 (BASF), GB Patent application 2073132 and WO 98/28396.

The use of such fragranced aminoplast capsules in liquid formulations for household and cosmetic applications is also well-known. For example, US Patent 5,188,754, assigned to Procter & Gamble, describes detergent compositions which contain perfumes in the form of friable microcapsules. US Patent 5,137,646, also to Procter & Gamble, describes the preparation and use of perfumed aminoplast particles which are stable in fluid compositions such as fabric softeners. However this composition demands a two-step manufacturing process where the perfume is first solidified with a meltable polymer followed by grinding of the solidified perfume and coating with the aminoplast resin.

It is also described that cationic transfer agents drive the deposition of such aminopast capsules on fabric, skin and hair. This is of particular importance when such aminoplast capsules are used in liquid rinse-off formulations like laundry detergents, fabric conditioners, shampoos, rinse-offhair conditioners and body washes.

US Patent 4,234, 627, assigned to Procter & Gamble, discloses a liquid fragrance coated with an aminoplast shell further coated by a water insoluble meltable cationic coating in order to improve the deposition of capsules from fabric conditioners. In US 4,973,422 (P&G), from 1989, it was then further described that capsules with a cationic coating provide improved substantivity to the surface being treated, such as fabric treated with a fabric softener. The same idea was described in 1991 in US 5,185,155, assigned to Unilever, where the selection of cationic polymers was enlarged to water soluble polymers and the type of encapsulation was distinct from those in the state of the art at the time. Patent application US 20040071742, assigned to IFF, discloses a similar technology where the fragranced aminoplast capsules are coated with cationic starch or cationic guar. International patent application WO 03/002699, assigned to Colgate-Palmolive, describes fabric softening compositions where a cationic cross-linked polymer improves deposition of friable aminoplast microcapsules. The improved deposition of cationic microcapsules in rinse-off formulations is also generally disclosed in US Patent application 2003/0171246, assigned to BASF, and International patent application WO 01/62376, assigned to Henkel.

However, as previously mentioned, the major down-side of the use of aminoplast capsules in rinse-off applications is that the perfumed capsules are not stable in such liquid formulations. The perfume gets extracted out off the capsules by surface active ingredients in the formulation. So far, none of the aminoplast capsules described heretofore remain stable for 2 months at 45°C in rinse-off formulations, i.e. under storage conditions that are encountered in many practical circumstances.

Some attempts have been made to increase the stability of such fragranced aminoplast capsules by modifying the capsules' membrane. For example, International patent application WO 02/074430, to Quest International, which outlines the above-mentioned stability problems of aminoplast capsules in aqueous surfactant-containing products, suggests a solution based on the use in the capsule shell of a second polymer comprising a polymer or copolymer of one or more anhydrides. It particularly describes improved stability in hair shampoo of aminoplast capsules prepared in the presence of ethylene(maleic anhydride) copolymer. While this solution improves the resistance of the capsules to degradation, it also prevents, as a consequence of the protection provided, the active ingredient, namely the perfume, from being easily released from the capsules. Therefore, these modified capsules require more energy than the original ones for the release of perfume, and this can constitute a drawback for their use in application. Moreover, the resulting stability after 1 month at 37°C is not sufficient for these applications.

On the other hand, WO 01/94001, assigned to Syngenta Ltd., mentions the possibility of having a solid permeable shell of a polymer resin having surface modifying compounds capable of reacting with isocyanate incorporated therein. While being based on the use of melamine resins, the latter shells have been modified so as to become permeable and, as a consequence, are susceptible of loosing the perfuming ingredients there-encapsulated through a diffusion process during the storage of the capsules.

EP0612843 describes a twin compartment pack containing a weak organic acid and a nonionic surfactant in one compartment and a granulated strong organic acid and a micro-encapsulated perfume in the second compartment.

US2003/0134765 relates to unit dose automatic dishwashing compositions, which could be in the form of a two compartment pouch containing a liquid composition in one compartment and a solid composition in the other compartment. Both the liquid and solid composition comprise surfactants. Further, a perfume is present in the solid composition.

Therefore, the solutions provided until now by the prior art are not satisfactory.

Now, the present invention provides a very cost effective and efficient solution to the problem related to the use of fragranced capsules based on aminoplast microcapsules, in melamine resin based microcapsules, or on coacervate capsules, in a surfactant-containing medium, while taking advantage of the release characteristics of these types of capsules. The solution of the invention consists of a packaged product comprising a physical separation between the fragranced aminoplast or coacervate capsules and the other ingredients of the product, in particular surfactants, thus keeping them separated during any desired storage time. This packaged product further allows a simple admixture of the contents of each separated compartment before or during use, thus providing a ready-to-use functional fragranced product, taking advantage of the encapsulated form of the perfume without encountering stability problems of the said capsules in the surfactant-containing base.

### Disclosure of the Invention

A first object of the invention is a packaged product comprising a container, a first component formed of a liquid base containing from 0.1 to 90% by weight, relative to the total weight of the base, of a surfactant system; and a second component formed of a perfuming base containing fragranced aminoplast microcapsules, namely melamine resin capsules, or fragranced coacervate capsules; wherein the container comprises at least two physically separated compartments, the first component and the second component being packaged in separate compartments. The packaged product according to the invention advantageously allows to submit the product to a prolonged storage without risking any alteration of the second component containing the reactive fragranced capsules, namely the melamine resin or coacervate capsules. Before using the product, the consumer can simply admix the contents of the separated compartments and then efficiently use the unified composition consisting of a mixture of a perfuming base and a treating base.

A further aspect of the invention concerns a method for treating a surface such as a textile, or yet skin or hair, using a packaged product as defined above, which method comprises the steps of mixing the contents of the separated compartments of the container and then applying the unified composition onto the surface to be treated.

Melamine resin and coacervate capsules both present many advantages when used as encapsulating systems for perfuming ingredients in application, i.e. in consumer products. First of all, when they break during use, for instance by friction when used in a shampoo or shower gel type application, a fresh fragrance burst is perceived. On the other hand, when especially treated, these capsules are susceptible of being efficiently laid down on a surface during their use in an application. Therefore, when a surface is treated with a product according to the invention, the perfumed capsules settle on said surface, typically a textile, the skin or yet hair, depending on the nature of the surfactant-containing component of the product, and provide an efficient release, by simple mechanical action such as a friction on the surface, of the perfuming ingredients encapsulated therein. These effects were not obtainable until now as these type of reactive capsules were altered by the surfactant-containing base during storage, before their possible use.

Within the framework of the invention the first component is formed of a surfactant-containing liquid base. The latter has a treating or cleaning activity, and may thus also be referred to as "treating base". More particularly this "treating base" designates a composition the formulation of which is specifically designed to provide a specific functional activity related to the treatment of a surface. Such an activity may consist for instance of a cleaning, purifying, detergent, softening, bleaching or other activity adapted to the treatment of a synthetic surface or for the treatment of skin or hair. The formulation of such treating bases comprises ingredients of varied nature and functions, depending on the treatment one wants to achieve with the product in question. Nowadays, the range of functional products and formulations has become so extensive and subjected to such frequent changes that a description based on a product-by-product approach and on the definition, for each case, of the composition of the treating base is impractical and would not be exhaustive anyway. However, a person skilled in the art is capable of choosing, without undue effort, suitable ingredients for the preparation of such a treating base designed for a particular purpose. What all such bases have in common is the presence of surfactants or surface active ingredients that are able to extract perfume out of fragranced microcapsules.

Many examples of such "treating bases" can be found in art textbooks and other literature, including the patent literature, relating to liquid consumer products commonly perfumed and which may in particular resort to the use of perfume capsules of the melamin or coacervate type. Such examples of typical literature are namely cited in the introduction above, but it goes without saying that the consumer products according to the invention can assume any form or formulation desired, provided that they contain a surfactant or surface active system which rendered prior known dispersions of fragranced aminoplast capsules, namely melamine resin, or coacervate capsules, unstable as far as the release of the perfume contained therein, or their stability in the dispersion, was concerned. This includes alcohols, alkylated glycols like propylene glycol or hydrophobic molecules modified by polyalkylated glycols like dimethicone copolyols or non-ionic emulsifiers of the Tergitol® type (ethoxylated fatty alcohols from Union Carbide).

Therefore, the packaged product of the invention is a ready-to-use perfumed consumer product with a treating or cleaning activity, typically a hair rinse-off preparation, a shampoo, a shower gel a liquid detergent for laundry or household, a liquid textile softener or yet a deodorant for laundry.

The treating base is characterised by the presence in its composition of a surfactant system comprising at least one surfactant or surface active ingredient, in a proportion varying between 0.1 and 90% by weight relative to the total weight of the base. The surfactant system used can be anionic, cationic, non ionic as well as amphoteric. However, there will be usually used a mixture of these types of surfactants. For example, for shampoo or shower gel applications, a combination of lauryl sulfate or laurylethersulfate with an amphoteric surfactant of the betaine type is common. For textile softeners, pure cationic surfactants such as distearyldimethylammoniumchloride or alkylammoniummethosulfate are typically used. On the other hand, household cleaners are often based on linear alkylsulfates (LAS), eventually in combination with non-ionic ethoxylated surfactants. To improve wetting of the treated surface and in order to reduce viscosity of the formulation, often alcohols and glycols like propylene glycol are added. These surfactant systems are given by way of example and shall not be taken to limit the invention, the person skilled in the art being quite capable of extending the principles of the instant invention to other surfactant systems of current use without undue effort. It goes without saying that the nature of the surfactant is quite immaterial for the practising of the invention, the objective of which is to improve upon any consumer product comprising fragranced melamine resin or coacervate capsules and a surfactant in which such capsules are not stable, i.e. any surfactant or surface active ingredient which causes partial or total extraction of the fragrance contained in such capsules when put in contact therewith. Any consumer product containing at least one such surfactant in an amount susceptible of causing such lysing of the capsules is therefore encompassed by the instant invention and hereby included.

The packaged product of the invention further comprises a second component formed of a perfuming base containing fragranced capsules that are not stable in the presence of the surfactant system.

In a first embodiment of the invention, the fragranced capsules consist of melamine resin microcapsules. These microcapsules are conventionally prepared by a polycondensation process comprising emulsifying the ingredient to be encapsulated, namely a perfuming ingredient or composition, in an aqueous solution of a melamin/formaldehyde resin, and then hardening the thus formed microcapsules. By "aqueous", it is meant here a composition essentially water-based but which may also contain other solvents compatible with the capsules such as for example ethanol.

Suitable capsules for the purpose of the invention are commercially available by manufacturers such as BASF (under the tradename of Micronal^{®}). Typically, these capsules will encapsulate from 20 to 85% by weight of perfume, relative to their total weight. Preferably, fragranced melamine resin capsules will be used in the form of a liquid aqueous suspension or dispersion. However, the capsules may also be used in the form of a dried powder, obtained after a drying treatment of a liquid suspension, e.g. via a spray-drying treatment.

In a second embodiment of the invention, the perfuming base essentially consists of fragranced capsules prepared by a simple or complex coacervation process. Simple coacervation involves the use of a single protein to form a capsule wall as phase separation is taking place. The complex process designates the use of a second oppositely charged non-protein polymer to bring about phase separation. Both methods are widely practiced in commercial processes and have been well described in the literature, namely in textbook works such as Capsule Technology and Microencapsulation, Chapter 4, M. Gutcho, New Jersey, 1972. Typically, these capsules have a perfume load comprised between 20 and 85% by weight relative to their total weight.

In both cases the concentration of capsules in the aqueous suspension or dispersion contained in one of the compartments of the packaged product will be typically comprised between 0.5 and 10% by weight, more preferably from 0.5 to 5% by weight, relative to the total weight of aqueous suspension.

The nature of the fragrance contained in the capsules is immaterial in the context of the invention, provided that it is compatible with the materials forming the capsules. It will be typically chosen as a function of the perfuming effect that is desired to achieve with the dispersion or consumer product of the invention, and it will be formulated according to current practices in the art of perfumery. It may consist of a perfume ingredient or composition. These terms can define a variety of odorant materials of both natural and synthetic origin, currently used for the preparation of perfumed consumer products. They include single compounds or mixtures. Specific examples of such components may be found in the current literature, e.g. Perfume and Flavor Chemicals by S. Arctander 1969, Montclair, N.J. (USA). These substances are well known to the person skilled in the art of perfumeing consumer products, i.e. of imparting an odor to a consumer product traditionally fragranced, or of modifying the odor of said consumer product.

Natural extracts can also be encapsulated into the system of the invention; these include e.g. citrus extracts such as lemon, orange, lime, grapefruit or mandarin oils, or essentials oils of plants, herbs and fruits, amongst other.

In addition to the fragranced capsules, the perfuming base of the invention may comprise optional ingredients such as antibacterial agents, thickeners, cosmetic emollients, vitamins, cooling agents, softeners, lubricants or any other active ingredient used for cosmetic or household applications, as long as the latter do not alter the capsules.

According to an advantageous embodiment of the invention, the aqueous dispersion of the capsules further comprises a polymeric thickening system. Such thickening polymers may be selected from non-ionic, anionic and cationic polymers. Preferably, a mixture of non-ionic and cationic polymers, in comparable weight amounts, shall be used.

Such systems of water soluble or water dispersible polymers can help stabilise the fragranced aminoplast capsules in liquid household and cosmetic formulations, which typically comprise water, silicone oils, organic and mineral oils, glycols and glycerin, by preventing separation of the capsules, particularly in low viscosity formulations, without affecting the flowability, spreading capability or sprayability of the liquid formulation.

Non-ionic polymers efficiently stabilise aqueous dispersions of aminoplast capsules at high viscosity. Non-ionic polymers susceptible of being used according to the invention include guar gums, hydroxyalkyl cellulose derivatives (for example, Tylose® from Shin Etsu and Clariant, Klucel® HF from Aqualon and Natrosol® from Hercules), carrageenan (namely from Kelco), cellulose, starch, maltodextrines, polymeric sugar derivatives, xanthan, PVP/VA copolymers (namely Luviskol® VA from BASF).

Anionic polymers appear to be the best stabilizers for liquid dispersions of anionic aminoplast capsules. The dispersions are stable even at low viscosity because they are stabilized by electrostatic charge. Repulsive action between equal charges supports the stability of the dispersion. Suitable anionic polymers comprise gum arabic, carboxymethylcellulose, (for example, Blanose® from Aqualon), anionic polyacrylates and alkylated copolymers (for example Carbopol® from Noveon and Salcare® AST from Ciba, Luviflex® Soft from BASF), ammonium polyacryloyldimethyl taurate (Hostacerin® AMPS from Clariant) and copolymers thereof.

The complex formation between anionic aminoplast capsules and cationic polymers is highly desired to drive the deposition of the capsules from rinse-off formulations onto the surfaces on which the latter are applied. Therefore, such cationic polymers are advantageously added to fragranced aminoplast capsules. Suitable cationic polymers comprise gelatine, quaternized hydrolisates of proteins (for example Gluadin® WQT from Cognis); polyquaternium polymers as cited in the CTFA Cosmetic Ingredient Dictionary, like cationic cellulose derivatives (Merquats® 100 and Ucare® JR 30 M from Amerchol), cationic guars (Cosmedia® Guar from Cognis), quaternized guars (Jaguar® C-162 from Rhodia) cationic polyacrylates (Salcare® SC 60 and Salcare® Super 7 from Ciba, or Eudragit® RL 30D from Röhm), cationic acrylamides (Rheovis® CDE from Ciba), polyquaternized polymers (like Luviquat® Care from BASF), polyethylene imine (Lupasol® P from BASF), quaternized polysiloxanes and emulsion polymerized aminosilicones (Abil® Quat 3270 from Degussa, Formasil® 410 from General Electric).

Although all of the above-mentioned polymers may be used on their own according to the invention as improving components of the aqueous dispersion of the capsules, we have found that combinations of non-ionic polymers and cationic polymers provided the best stability of the dispersion of aminoplast capsules in liquid rinse-off formulations. The advantage of this preferred embodiment of the invention is that the polymer combination does not cause agglomeration and separation of the capsules and does not have a negative impact on the deposition effect of the cationic polymer on the anionic aminoplast capsule. The non-ionic polymer protects the aminoplast capsule from agglomerating with the cationic polymer and the latter keeps the aminoplast capsules well dispersed even in low-viscosity formulations. This is for example the case when such formulations are stored at elevated temperature, i.e. at 40 - 50°C. Many non-ionic polymer thickeners loose their thickening efficiency and the viscosity of the entire formulation is reduced. In this case, the aminoplast capsules start separating if there is no cationic polymer present.

With a combination of non-ionic and cationic polymers, the total viscosity of the liquid formulation can be kept very low such that the formulation is easy to spray and spreads well on surfaces such as skin, hair and fabric, or household surfaces. This preferred embodiment of the invention thus improves on the embodiments which resort to the use of only one type of thickening polymer as described above.

Typical concentrations of thickeners in the above embodiments of the invention vary from 0.1 to 10%, preferably from 1 to 5% by weight, relative to the weight of the composition. When combinations of non-ionic and cationic polymers are used, the relative proportions of the latter are comprised in a range of 5:1 to 1:5. Preferably, comparable weight amounts of non-ionic and cationic polymer shall be used.

The packaged product of the present invention provides a solution to the problem of instability of aminoplast or coacervate capsules in the presence of surfactants by providing physical separation between the capsules and the surfactant-containing treating base. It goes without saying that the product of the invention is suitable for other types of polymeric capsules which encounter the same diffusion problems in the presence of a surfactant system as melamine resin or coacervate capsules. Reactive delivery systems encapsulating flavour ingredients could for instance also be considered.

The packaged product according to the invention is characterised by the fact that the first component, namely the surfactant-containing liquid base on the one hand, and second component, namely the perfuming base on the other, are held in separate compartments of the container. Typically, both compartments will be of more-or-less comparable volume. The compartment with the capsules will typically contain aqueous dispersions of from 0.1% to 8% weight of capsules, relative to the total weight of the aqueous dispersion.

The container may be made of various materials. Typically, it is made of plastics such as PET, OPP, PE or polyamide and including mixtures, laminates or other combinations of these. One can also manufacture a packaging formed of water soluble plastics like polyvinyl alcohol and, if the water in the liquid formulation is replaced by glycerin, propylene glycol, liquid surfactants or other non-aqueous liquids.

Different embodiments may be considered as regards the physical separation of the compartments of the container.

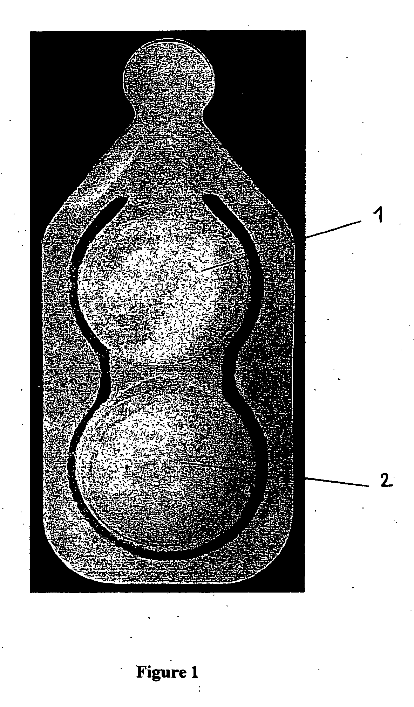

In a first embodiment, as represented in Figure 1, the separate compartments have a common joint formed by at least two sheets of polymeric material, welded or laminated with a breakable seal which ruptures internally along a longitudinal axis, without the external walls of the packing rupturing, when an external pressure is applied to one of the compartments. This embodiment enables to mix up the contents of the separated compartments just before use, after rupture of the internal portion of the joint separating said compartments. The sheet of polymeric material which ruptures internally along a longitudinal axis under the action of an external pressure is made of a polymeric material of the foam type and constitutes a breakable seal. Materials of this type include in particular polyethylene, polypropylene, polyamides, polyvinyl chloride and polyesters. These are preferably provided in the form of sheets having a thickness of between 0.005 and 0.2 mm. In this embodiment, the packaged product of the invention can be in the form of a monodose of 2 or more parts or blisters which are stored separately and mixed together prior to use, by applying a finger pressure on the outside of one blister to break the seal and mix the two bases.

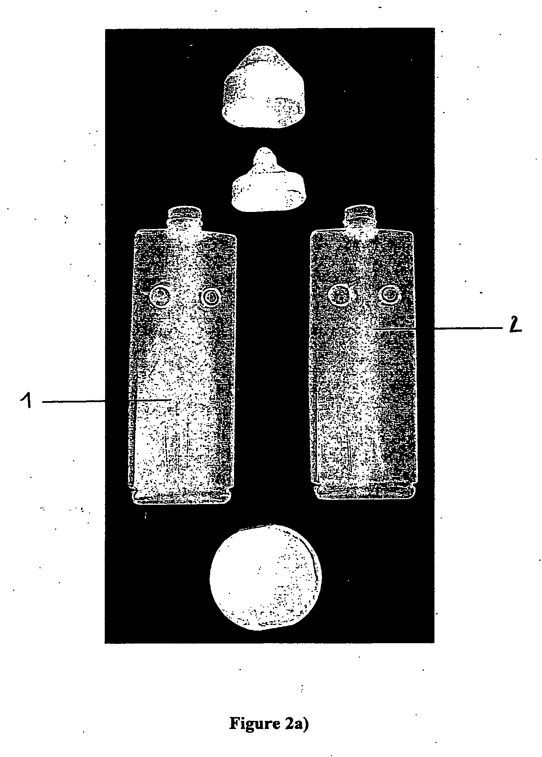

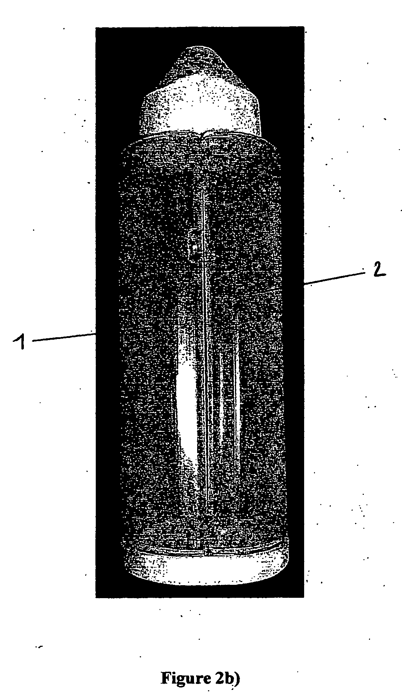

In a second embodiment of the invention illustrated in Figure 2a), the treating base on the one hand and the perfuming base on the other hand are held in separate bodies, for instance having the shape of half cylinders, provided with a neck and an opening, and which can be arranged and fixed side by side as shown in Figure 2b). In this embodiment, there is no breakable seal between the two compartments, but the contents of the latter can simply be mixed before use, during outpouring, as the outlet piece unifies the two liquids coming from the separate compartments.

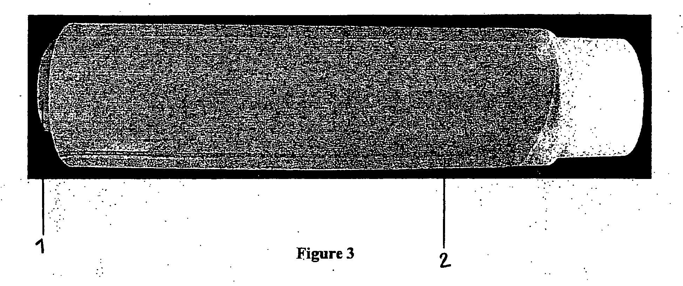

In another embodiment, as shown in Figure 3, the separate bodies are two cylinders forming a tube, the first one with a smaller diameter than that of the second one and located inside the second one, so that the outlets are formed by two concentric openings (of any shape) and allow the mixing of the contents of the compartments upon use of the product when the two bases are squeezed out.

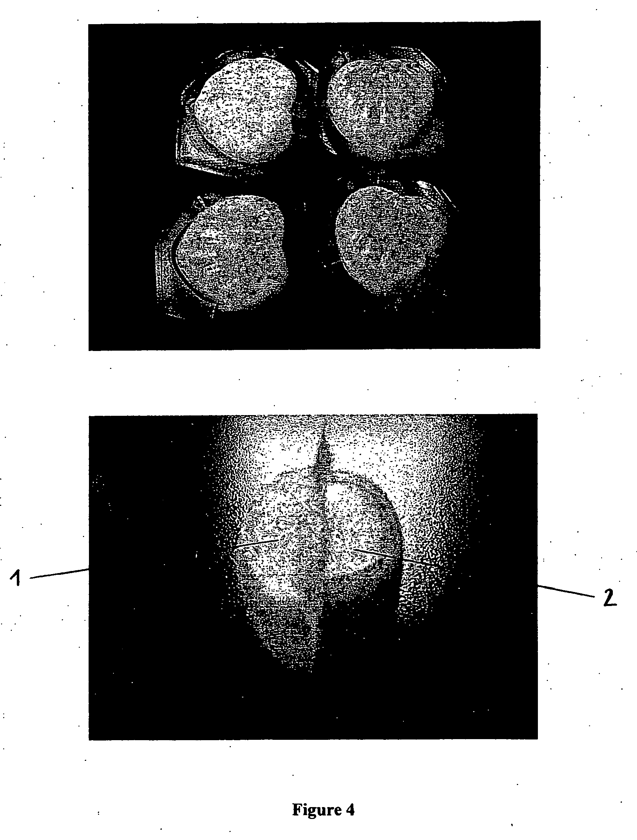

The packaged product of the invention can also be made of a water-soluble foil, as shown in Figure 4, and have two compartments separated by a breakable or water soluble seal, wherein the first component is a water-free liquid surfactant-containing base, and the second one is a perfuming base containing reactive fragranced capsules. When used in an aqueous environment, the packaged product dissolves and the contents of the compartments are therefore combined upon use of the product.

The invention is not limited to these embodiments, as many different ways of preparing two separated compartments assembled together in a way that allows their contents to be combined before use of the product may be considered and suit the invention.

The invention will now be described in greater detail in the following examples wherein the temperatures are indicated in degrees Celsius and the abbreviations have the usual meaning in the art.

### Brief Description of the Drawings

Figure 1 illustrates a mono-dose or single dose packaged product according to the invention, wherein a first compartment (part 1) comprises fragranced capsules in the form of an aqueous suspension, while a second compartment (part 2) comprises a liquid detergent base. The upper and lower support of part 1 are made of a foam breakable seal while the lower support of part 2 is made of a solid un-breakable seal.
Figure 2a) illustrates a plastic bottle with separated compartments, wherein a first compartment (part 1) comprises fragranced capsules in the form of an aqueous suspension, while a second compartment (part 2) comprises a liquid detergent base.
Figure 2b) shows the closed bottle wherein the two compartments have been assembled together.
Figure 3 illustrates a plastic tube made of 2 cylindrical bodies, the first one (part 1) with a smaller diameter than the second one (part 2) and located inside the second one.
Figure 4 illustrates a mono-dose or single dose packaged product according to the invention, wherein a first compartment (part 1) comprises fragranced capsules in the form of an aqueous suspension, while a second compartment (part 2) comprises a liquid detergent base. The two liquids can be unified by cutting a sealed edge between the two compartments or, in case of water soluble plastic, by placing the entire packaging in water.

### Modes of Carrying out the Invention

### Example 1

### Mono-dose shampoo

A mono-dose packaging illustrated in Figure 1 was prepared as follows : a thermoformed blister was made of a polypropylene foil of 200 µm thickness. The cover foil was a laminate of 20 µm polyamide and 20 µm of a peel layer forming a breakable seaL

Part 1 of the packaging represented in Fig. 1 was charged with the following composition:

| | |
|---|---|
| 5 g | of an aqueous suspension of fragranced capsules prepared by conventional coacervation process of gelatin with gum Arabic. The capsules contained 40% of lemon perfume oil and 40% of a skin emollient; |
| 30 g | of water thickened with 0.5% Natrosol^{®} 250H |
| 0.08 g | of Kathon^{®} CG (methylchloroisothiazolinone and methylisothiazolinone ; origin: Röhm & Haas, USA) |

Part 2 of the packaging represented in Fig. 1 was filled with 30 g of the following shampoo base, prepared as described here-below:

| *Ingredients* | *Parts by weight* |
|---|---|
| Texapon^{®}NSO IS¹⁾ | 63.00 |
| Comperlan^{®}KD²⁾ | 3.00 |
| Deionised water | 31.36 |
| Sodium chloride | 0.50 |
| Citric acid | 0.04 |
| Katon^{®}CG ³⁾ | 0.10 |
| Perfume ⁴⁾ | 2.00 |
| Total | 100.00 |

| | |
|---|---|
| 1) sodium laureth sulfate ; origin : Henkel 2) cocamide DEA; origin: Henkel 3) methylchloroisothiazolinone and methylisothiazolinone; origin: Rohm & Haas 4) origin : Firmenich SA, Geneva, Switzerland | |

Texapon^{®}NSO IS and Comperlan^{®}KD were mixed together. Seperately, the sodium chloride and the Kathon^{®}CG were dissolved in the distilled water, then citric acid was added.

The latter mixture was poured little by little into the first one, while mixing slowly in order to avoid incorporating air. The final viscosity was regulated with sodium chloride and pH was adjusted to 6.0 with citric acid.

A simple finger pressure on part 2 of the packaged product allowed the unification of the shampoo base and the perfuming capsules. The combined composition provided a ready-to-use mono-dose perfumed shampoo.

The shampoo was tested on a hair lock which was conventionally washed with the product. During washing of hair, the capsules were broken and a fresh fragrance burst was liberated, in addition to the standard fragrance used in part 2. After rinsing, and drying, it was observed that when touching and slightly rubbing the hair, some perfume was still released in the form of a burst of perfume. This showed that the perfumed capsules were not altered and had efficiently settled on the hair surface treated, thus allowing an efficient and prolonged perfume release.

### Example 2

### Packaged textile softener

Two half cylinders as represented in Figure 2a) were prepared by blow extrusion of high density polyethylene.

Part 1 of the packaging represented in Fig. 2a) was charged with the following composition:

| | |
|---|---|
| 2 g | of an aqueous suspension of fragranced Micronal^{®} melamine resin capsules (origin: BASF, Germany) containing 80% of perfuming base (origin: Firmenich SA, Switzerland) |
| 28 g | of water thickened with 0.8% Jaguar^{®} C62 (origin: Rhodia, France) |
| 0.05 g | of Kathon^{®}CG (methylchlomisothiazolinone and methylisothiazolinone; origin: Röhm & Haas, USA) |

Part 2 of the packaging represented in Fig. 2a) was filled with 50 g of the following emulsion, prepared as described below:

| *Ingredients* | *Parts by weight* |
|---|---|
| Stepantex^{®} VS 90 ¹) | 16.50 |
| Calcium chloride solution at 10% | 0.20 |
| Dye (1% aqueous solution) | 0.30 |
| Fragrance ²) | 1.00 |
| Water deionised | 82.00 |
| Total | 100.00 |

| | |
|---|---|
| 1) dialkylammoniummethosulfate ; origin: Stepan, France 2) origin: Firmenich SA, Switzerland | |

The Stepantex^{®} was slowly poured into the water under stirring and at 40-50°, and further stirred for 10 min until the emulsion was homogeneous. The calcium chloride solution was added by portions during the addition of Stepantex^{®} to avoid the formation of a gel. The dye and perfume were added around 35° and the mixture was stirred for another 5 min.

The two compartments were assembled and fixed side by side as shown in Fig. 2b). During outpouring, the outlet piece unified the two liquids coming from the separate cylinders, thus forming an efficient perfumed textile softener providing for the transfer of intact capsules onto a textile upon treatment of the latter with the softener.

### Example 3

### Self foaming shower gel

A mono-dose package as described in Example 1 was used.

Part 1 of the packaging represented in Fig. 1 was charged with the following composition :

| | |
|---|---|
| 5.0 g | of an aqueous suspension of fragranced melamine resin capsules. The capsules contained 80% of concentrated perfume |
| 43.0 g | of demineralised water |
| 3.5 g | of crystalline citric acid |
| 0.5 g | of Natrosol^{®} 250H |

Part 2 of the packaging represented in Fig. 1 was filled with 50 g of the following clear shower gel base, prepared as described below :

| | *Ingredients* | *Parts by weight* |
|---|---|---|
| A) | Texapon^{®} NSO IS¹⁾ | 55.10 |
| | Comperlan^{®} KD ²⁾ | 3.00 |
| B) | Deionised water | 31.40 |
| | Sodium hydrogen carbonate | 8.40 |
| | Katon^{®} CG³⁾ | 0.10 |
| | Perfume⁴⁾ | 2.00 |
| | Total | 100.00 |

| | | |
|---|---|---|
| 1) sodium laureth sulfate ; origin : Henkel 2) cocamide DEA; origin : Henkel 3) methylchloroisothiazolinone and methylisothiazolinone ; origin : Rohm & Haas 4) origin: Firmenich SA, Geneva, Switzerland | | |

Texapon^{®} NSO IS and Comperlan^{®} KD were mixed together. In another container, part B formed by the sodium hydrogen carbonate and the Kathon^{®} CG, was dissolved in the distilled water.

The latter mixture was poured little by little into the first one (A), while mixing slowly in order to avoid incorporating air. Finally, the liquid perfume was added.

For activation, the pouch of part 2 was pressed to break the separation seal. When the two parts were mixed, the CO₂ (formed by the reaction of sodium hydrogen carbonate and citric acid) developed foam and pressure. This burst the breakable seal on the top of the mono-dose packaging and expelled the foam. The unified contents were then ready for use. The fragranced melamine resin capsules were broken during the friction on the skin and liberated a fresh fragrance burst during use. Moreover, some intact capsules remained on the skin or in the hair and liberated fresh perfume odour when the dry skin or hair was rubbed or combed later during the day.

### Example 4

### Water-soluble container for laundry detergent

A liquid concentrated laundry detergent was packed with fragranced melamine resin microcapsules in the form of a spray-dried powder, in water-soluble polyvinyl alcohol sheets or bags.

Part 1:0.5g of capsules having the composition disclosed in Example 2, further subjected to a conventional spray-drying process to form a spray-dried powder.

Part 2 : 25 g of water-free liquid detergent based of the following composition:

| *Ingredients* | *Parts by weight* |
|---|---|
| Linear alkylsufonate | 5.00 |
| Fatty alcohol ethoxylate 7EO | 74.00 |
| Polyglycol 400 | 20.50 |
| Citric acid | 0.50 |
| Total | 100.00 |

Part 1 and part 2 were filled separately in a two compartment bag made of a water soluble polyvinyl alcohol sheet of 80 mm thickness (Soltec^{®} T10; origin: Soltec, Paris). When dissolved in water, the two parts were combined upon use.

### Example 5

### Compositions comprising fragranced melamin capsules

The following compositions, intended to be used as the component comprising the fragranced capsules contained in one of the separate compartments of the packaged product, were prepared by admixture of their ingredients and compared amongst themselves for stability of the capsule suspension in the aqueous solution.

| Ingredient | Composition/ | | | | |
|---|---|---|---|---|---|
| | % by weight | | | | |
| | I | II | III | IV | V |
| fragranced melamin resin capsules, 40% by weight dispersion in water | 4.0 | 4.0 | 4.0 | 4.0 | 4.0 |
| water | q.s. to 100 | q.s. to 100 | q.s. to 100 | q.s. to 100 | q.s. to 100 |
| Tylose®, Mw 60'000, 2% aqu. solution | 40 | | 40 | | |
| Salcare® SC60, 2% aqu. sol. | | 40 | 40 | | |
| Keltrol® SF, 2% aqu. solution | | | | 40 | |
| Hostacerin® AMPS, 2% aqu. solution | | | | | 40 |
| Stability after 1 month, 45°C | some separation observed | some agglomeration and separation occurred | stable | stable but some fragrance leakage from capsules | stable |

In the packaged product of the invention, the two compartments are typically of more-or-less equal volume. Hence the fragranced capsules need to be diluted in order to obtain equal volume compared to the surfactant system in the other compartment.

The compartment with capsules typically contains aqueous dispersions comprised from 0.1% to 8% weight of capsules.

It was found that aqueous dispersions of fragranced capsules such as Micronal® are not stable as such. The capsules require a thickening system to remain dispersed.

As the table above shows, non-ionic thickeners such as Tylose® can stabilize such dispersions at ambient temperature, but at 45°C many non-ionic thickeners start thinning and allow some separation. To avoid such separation, the thickeners need to be dosed at such high concentration that the resulting dispersion has a creamy viscosity. For usage in the two-compartment packaged product of the invention, such high viscosity is not desirable.

In the case of anionic thickeners, such as Keltrol® SF, improved stability at high temperatures could be obtained, but such thickeners may cause some release of the fragrance from the capsules during ageing. When AMPS polymers and copolymers were used, like Hostacerin® AMPS or Aristoflex® AVC, no release of fragrance was observed.

Cationic polymers, such as Salcare® SC60, Cosmedia® Guar or different types of Jaguar®, do not affect the capsule stability but may promote agglomeration of anionic Micronal® capsules, resulting in some separation of the dispersion.

As the table shows, it was now found that aqueous dispersions of anionic melamin/formaldehyde capsules can be best stabilised by combinations of non-ionic thickeners and cationic thickening polymers. The capsules are first dispersed in a solution of the non-ionic thickener. To this dispersion, the cationic thickening polymer is added.

This procedure avoids agglomeration of the anionic capsules when they come into contact with the cationic thickening polymer. The cationic polymer enhances dispersion stability of the capsules at elevated temperature without extracting the encapsulated fragrance.

It was now found that aqueous dispersions of anionic melamin/formaldehyde capsules can be stabilized by combinations of non-ionic thickeners and cationic thickening polymers.

It was also found that anionic thickeners of the AMPS type and copolymers thereof provide excellent stability of anionic melamin/formaldehyde capsules without extracting perfume from the capsules.

All the compositions made it possible to the stability and homogeneity of the aqueous dispersion of fragranced melamin capsules, composition III and V having provided the best stability during storage, under the conditions indicated.

## Claims

1. A packaged product comprising:
a) a container;
b) a first component formed of a liquid base containing from 0.1 to 90% by weight, relative to the total weight of the base, of a surfactant or surface active ingredient system; and
c) a second component formed of a perfuming base comprising fragranced melamine resin capsules or fragranced coacervate capsules ;
wherein the container comprises at least two physically separated compartments, the first component and the second component being held in separate compartments.

2. A packaged product according to claim 1, wherein the second component comprises an aqueous suspension of fragranced melamine resin microcapsules.

3. A packaged product according to claim 1, wherein the two compartments comprise a common joint formed by at least two sheets of polymeric material, welded or laminated with a breakable seal which ruptures internally along a longitudinal axis without the external walls of the package rupturing, when an external pressure is applied to one of the compartments.

4. A packaged product according to claim 1, wherein the two physically separated compartments each comprises an opening located close to one another in a manner such as to allow unification of the first and second components upon use.

5. A packaged product according to claim 1, wherein the two physically separated compartments are made of a water-soluble foil or film and are physically separated by a breakable seal.

6. A packaged product according to claim 1, wherein the first component is selected from the group consisting of a liquid detergent base, a fabric softener base, a shampoo, a rinse-off hair conditioner base, a shower gel base, a foam bath gel base, and a liquid all purpose cleaner base.

7. A packaged product according to claim 1, wherein the second component comprises a thickening polymer system.

8. A packaged product according to claim 7, wherein the thickening polymer system is a mixture of non-ionic and cationic polymer.

9. A packaged product according to claim 7, wherein the thickening polymer system is an anionic polymer of AMPS type or copolymers thereof.

10. A packaged product according to claim 1 or 7, wherein the second component comprises an aqueous suspension containing from 0.5 to 8% by weight of fragranced capsules, relative to the weight of the suspension.

11. A packaged product according to claim 7, 8 or 9, wherein the thickening polymer system is present in a weight range of from 0.1 to 10% by weight, relative to the weight of the second component.

12. A method for treating a surface using a packaged product according to any one of claims 1 to 10, comprising the steps of mixing the contents of the physically separated compartments of the container and applying the combined contents of the components onto the surface to be treated.

13. A method according to claim 11, using a packaged product according to claim 3, wherein the contents of the separate compartments are combined by applying pressure on the outside of one compartment to induce the breaking of the seal separating the compartments.

14. Use of a container with multiple compartments for holding a first component formed of a liquid base containing from 0.5 to 90% by weight relative to the total weight of the base of a surfactant system; and a second component formed of a perfuming base comprising fragranced melamine resin capsules or fragranced coacervate capsules; wherein the first and second components are held in separated compartments and combined upon use of the container to treat a surface.

## Patentansprüche

1. Verpacktes Produkt, umfassend:
a) ein Behältnis;
b) eine erste Komponente, die aus einer flüssigen Basis gebildet ist, die von 0,1 bis 90 Gew.-%, bezogen auf das Gesamtgewicht der Basis, eines Systems aus einem Tensid oder einem oberflächenaktiven Bestandteil enthält; und
c) eine zweite Komponente, die aus einer Parfümbasis, umfassend parfümierte Melaminharz-Kapseln oder parfümierte Koazervat-Kapseln, gebildet ist;
worin das Behältnis mindestens zwei physikalisch getrennte Kammern umfasst, wobei die erste Komponente und die zweite Komponente in getrennten Kammern gehalten werden.

2. Verpacktes Produkt nach Anspruch 1, worin die zweite Komponente eine wässrige Suspension aus parfümierten Melaminharz-Mikrokapseln umfasst.

3. Verpacktes Produkt nach Anspruch 1, worin die zwei Kammern aus einer gemeinsamen Verbindung bestehen, gebildet durch mindestens zwei Folien aus Polymermaterial, verschweißt oder laminiert mit einer zerbrechlichen Abdichtung, die intern entlang einer Längsachse reißt, ohne dass die Außenwände der Verpackung reißen, wenn ein Außendruck auf eine der Kammern aufgebracht wird.

4. Verpacktes Produkt nach Anspruch 1, worin die zwei physikalisch getrennten Kammern jeweils eine Öffnung umfassen, die sich nahe zueinander auf eine Weise dergestalt befinden, dass die Vereinigung der ersten und zweiten Komponenten bei Gebrauch ermöglicht wird.

5. Verpacktes Produkt nach Anspruch 1, worin die beiden physikalisch getrennten Kammern aus einer wasserlöslichen Folie oder Feinfolie hergestellt sind und physikalisch durch eine brechbare Abdichtung getrennt sind.

6. Verpacktes Produkt nach Anspruch 1, worin die erste Komponente aus der Gruppe ausgewählt ist, bestehend aus einer flüssigen Detergensbasis, einer Gewebeweichmacherbasis, einem Shampoo, einer ausspülbaren Haarpflegespülungsbasis, einer Duschgelbasis, einer Schaumbadgelbasis und einer flüssigen Allzweck-Reinigungsmittelbasis.

7. Verpacktes Produkt nach Anspruch 1, worin die zweite Komponente ein Verdickungspolymersystem umfasst.

8. Verpacktes Produkt nach Anspruch 7, worin das Verdickungspolymersystem ein Gemisch aus nicht ionischen und kationischen Polymeren darstellt.

9. Verpacktes Produkt nach Anspruch 7, worin das Verdickungspolymersystem ein anionisches Polymer des AMPS-Typs oder Copolymere davon darstellt.

10. Verpacktes Produkt nach Anspruch 1 oder 7, worin die zweite Komponente eine wässrige Suspension umfasst, bestehend aus 0,5 bis 8 Gew.-% parfümierten Kapseln bezogen auf das Gewicht der Suspension.

11. Verpacktes Produkt nach Anspruch 7, 8 oder 9, worin das Verdickungspolymersystem in einem Gewichtsbereich von 0,1 bis 10 Gew.-% bezogen auf das Gewicht der zweiten Komponente vorliegt.

12. Verfahren zur Behandlung einer Oberfläche unter Verwendung eines verpackten Produkts nach einem der Ansprüche 1 bis 10, umfassend die Schritte des Mischens des Inhalts der physikalisch getrennten Kammern des Behältnisses und Aufbringen des kombinierten Gehalts der Komponenten auf die zu behandelnde Oberfläche.

13. Verfahren nach Anspruch 11 unter Verwendung eines verpackten Produkts nach Anspruch 3, worin der Inhalt der getrennten Kammern durch Aufbringen von Druck auf die Außenseite von einer Kammer zur Induktion des Brechens der die Kammern trennenden Abdichtung kombiniert wird.

14. Verwendung eines Behältnisses mit Mehrkammern zur Aufnahme einer ersten Komponente, die aus einer flüssigen Basis gebildet ist, enthaltend von 0,5 bis 90 Gew.-%, bezogen auf das Gesamtgewicht der Basis, eines oberflächenaktiven Systems; und einer zweiten Komponente, die aus einer Parfümbasis gebildet ist, umfassend parfümierte Melaminharz-Kapseln oder parfümierte Koazervat-Kapseln; worin die ersten und zweiten Komponenten in getrennten Kammern gehalten und bei Gebrauch des Behältnisses zur Behandlung einer Oberfläche kombiniert werden.

## Revendications

1. Produit emballé comprenant:
a) un conteneur;
b) un premier composant formé par une base liquide contenant de 0,1 à 90% en poids, par rapport au poids total de la base, d'un système surfactant ou d'ingrédient tensioactif; et
c) un second composant formé par une base parfumante comprenant des capsules de résine de mélamine parfumée ou des capsules de coacervat parfumé;
dans lequel le conteneur comprend au moins deux compartiments physiquement séparés, le premier composant et le second composant étant gardés dans des compartiments séparés.

2. Produit emballé selon la revendication 1, dans lequel le second composant comprend une suspension aqueuse de microcapsules de résine de mélamine parfumée.

3. Produit emballé selon la revendication 1, dans lequel les deux compartiments comprennent une jointure commune formée par au moins deux feuilles de matériau polymère, soudées ou laminées avec un sceau cassable qui se rompt intérieurement le long d'un axe longitudinal sans que les parois extérieures de l'emballage ne se rompent, quand une pression externe est appliquée sur l'un des compartiments.

4. Produit emballé selon la revendication 1, dans lequel les deux compartiments physiquement séparés comprennent chacun une ouverture située l'une près de l'autre de façon à permettre l'unification du premier et du second composants au moment de l'utilisation.

5. Produit emballé selon la revendication 1, dans lequel les deux compartiments physiquement séparés sont faits d'un feuillet ou film soluble dans l'eau et sont physiquement séparés par un sceau cassable.

6. Produit emballé selon la revendication 1, dans lequel le premier composant est sélectionné dans le groupe constitué par une base de détergent liquide, une base d'adoucissant pour tissu, un shampooing, une base d'après-shampooing à rincer, une base de gel douche, une base de gel bain mousse, et une base de nettoyant liquide universel.

7. Produit emballé selon la revendication 1, dans lequel le second composant comprend un système polymère épaississant.

8. Produit emballé selon la revendication 7, dans lequel le système polymère épaississant est un mélange de polymères non-ionique et cationique.

9. Produit emballé selon la revendication 7, dans lequel le système polymère épaississant est un polymère anionique de type AMPS ou des copolymères de celui-ci.

10. Produit emballé selon la revendication 1 ou 7, dans lequel le second composant comprend une suspension aqueuse contenant de 0,5 à 8% en poids de capsules parfumées, par rapport au poids de la suspension.

11. Produit emballé selon la revendication 7, 8 ou 9, dans laquelle le système polymère épaississant est présent dans un intervalle de poids de 0,1 à 10% en poids, par rapport au poids de second composant.

12. Méthode pour traiter une surface en utilisant un produit emballé selon l'une quelconque des revendications 1 à 10, comprenant les étapes de mélange des contenus des compartiments physiquement séparés du conteneur et d'application des contenus combinés des composants sur la surface à traiter.

13. Méthode selon la revendication 11, utilisant un produit emballé selon la revendication 3, dans laquelle les contenus des compartiments séparés sont combinés par l'application de pression sur l'extérieur d'un compartiment pour induire la rupture du sceau séparant les compartiments.

14. Utilisation d'un conteneur avec compartiments multiples pour tenir un premier composant formé par une base liquide contenant de 0,5 à 90% en poids par rapport au poids total de la base d'un système surfactant; et un second composant formé par une base parfumante comprenant des capsules de résine de mélamine parfumée ou des capsules de coacervat parfumé; dans laquelle le premier et le second composants sont tenus dans des compartiments séparés et combinés au moment de l'utilisation du conteneur pour traiter une surface.
